Europäisches Patentamt

European Patent Office

Office européen des brevets

(11) Veröffentlichungsnummer: **0 175 185**
**B1**

(12) **EUROPÄISCHE PATENTSCHRIFT**

(45) Veröffentlichungstag der Patentschrift: 05.12.90

(21) Anmeldenummer: 85110864.7

(22) Anmeldetag: 29.08.85

(51) Int. Cl.⁵: **A 61 K 35/78**

(54) Verfahren zur Gewinnung haltbarer wirkstoffreicher Kamillenextrakte.

(30) Priorität: 19.09.84 DE 3434342

(43) Veröffentlichungstag der Anmeldung:
26.03.86 Patentblatt 86/13

(45) Bekanntmachung des Hinweises auf die
Patenterteilung:
05.12.90 Patentblatt 90/49

(84) Benannte Vertragsstaaten:
AT BE CH DE FR IT LI LU SE

(56) Entgegenhaltungen:
EP-A-0 096 016
EP-A-0 154 872
DE-A-2 316 363
DE-B-1 093 951
FR-A-1 015 120

(73) Patentinhaber: ASTA Pharma Aktiengesellschaft
Weismüllerstrasse 45
D-6000 Frankfurt am Main 1 (DE)

(72) Erfinder: Isaac, Otto, Dr.
Liesingstrasse 8
D-6450 Hanau 9 (DE)
Erfinder: Carle, Reinhold, Dr.
Im Taubhaus 56 A
D-6074 Rödermark (DE)

**Beschreibung**

Kamillenfluidextrakte lassen sich auf verschiedene Weise gewinnen, beispielsweise durch Mazeration, Perkolation, Reperkolation oder Kettenperkolation. Allen Verfahren ist gemeinsam, daß sie von der Kamillendroge, das heißt den getrockneten Blütenköpfchen ausgehen. Für die Qualitätsbeurteilung von Kamillenblüten und Kamillenextrakten sind neben dem Bisabololgehalt die Gehalte an ätherischem Öl, Chamazulen und Flavonen maßgebend.

Durch den Trocknungsprozeß verlieren die Kamillenblüten 75—85% ihres Gewichtes. Gleichzeitig erleiden die Blüten aber auch einen Wirkstoffverlust, dessen Ausmaß von der Art der Trocknung abhängig ist. Bei der Trocknung einer Frischkamille vom Bisaboltyp unter fabrikationsmäßigen Bedingungen ist beispielsweise auch bei relativ niedrigen Trocknungstemperaturen noch mit einem Verlust von 47% äthermischem Öl, beziehungsweise 48% Chamazulen und 46% (−)-α-Bisabolol zu rechnen.

Weitere Wirkstoffverluste treten bei der Lagerung der Kamilliendroge ein. So wurde beispielsweise nach einjähriger Lagerung von Kamillendroge eine Abnahme des Gehaltes an ätherischem Öl von 43,8% beobachtet, wobei der Chamazulengehalt des ätherischen Öls sogar überproportional um 68,4% abgenommen hatte.

Hinzu kommt, daß die Kamillenwirkstoffe aus der Droge nur schwer extrahierbar sind. So geht beispielsweise bei der Herstellung der Fluidextrakte mit 45 %igem Alkohol nur etwa die Hälfte des in der Droge enthaltenen ätherischen Öls in den Extrakt über.

Eine Extraktion von Frischkamille (das heißt frisch geernteten Blütenköpfchen wurde bis jetzt nicht vorgenommen, da frisch geerntete Pflanzen oder Pflanzenteile zahlreichen enzymatisch gesteuerten oder mikrobiell verursachten Prozessen unterliegen, die eine Veränderung der Inhaltsstoffe zur Folge haben. Erst durch den mit der Trocknung erfolgenden Entzug des Wassers erfolgt eine Inaktivierung beziehungsweise Denaturierung der pflanzlichen Enzyme. Lediglich in Arch. Pharm. 280 (1942) Seite 437-38 wird ein Fall beschrieben, wo frische Kamille mit 86 %igem Alkohol extrahiert werden, wobei anschließend jedoch zur Zerstörung der Enzyme 20 Minuten zum Sieden erhitzt wird. Die Azulenausbeute betrug hierbei jedoch nur 18,4% und bereits nach 5 Monaten war praktisch kein Azulen mehr vorhanden.

Auch sind die Wirkstoffe in den bekannten Kamillenauszügen nur begrenzt haltbar. Insbesondere nimmt der Gehalt an Chamazulen schnell ab, was sich auch durch Zusätze von Alkalien nicht verhindern läßt.

Überraschenderweise hat sich nun herausgestellt, daß die erfindungsgemäße Frischkamillenextraktion gegenüber der bekannten Kamillendrogenextraktion Vorzüge aufweist. Insbesondere ist es möglich, die Wirkstoffausbeute und die Haltbarkeit der Wirkstoffe zu verbessern.

Wider Erwarten ist es dabei nicht erforderlich, den Frischpflanzenextrakt durch Erhitzen zu stabilisieren. Unbeschadet einer weiterbestehenden enzymatischen Aktivität muß ein Erhitzen des Frischkamillenauszuges sogar vermieden werden, um die beschriebenen Vorzüge zu sichern.

Die Erfindung betrifft die durch die Patentansprüche definierten Gegenstände beziehungsweise Sachverhalte.

Die Extraktion der frischen Kamillenblüten erfolgt mit gesättigten aliphatischen Alkoholen mit 1—4 C-Atomen. Als Alkohole kommen zum Beispiel in Frage: Methanol, Ethanol, Propanol-(1), Propanol-(2), insbesondere Ethanol und Isopropanol. Es können die reinen Alkohole (100%) verwendet werden oder Mischungen dieser Alkohole mit Wasser, wobei der Alkoholgehalt zwischen 40 und 100 Gewichts%, insbesondere 50—90, vorzugsweise 55—85 Gewichts% liegen kann.

Die Extraktion wird in einem Temperaturbereich zwischen 10°C und 60°C, vorzugsweise 10°C und 40°C durchgeführt.

Die Alkoholkonzentration im Extraktionsmittel richtet sich nach dem Drogenäquivalent oder dem Trockengewicht der Kamille, welches in dem resultierenden Extrakt vorliegt. Das Drogenäquivalent einer Frischkamille ist die Gewichtsmenge Kamillendroge, die man aus dieser Frischkamille durch die übliche Trocknung, das heißt wie sie zur Herstellung von Kamillendrogen üblicherweise durchgeführt wird, erhält, beispielsweise durch Trocknung in dünnen Schichten unter Ausschluß des direkten Sonnenlichts bei Lufttemperaturen zwischen 40—60°. Eine derartige Droge enthält im allgemeinen noch bis zu 10 Gewichts% Wasser. Das Trockengewicht einer Kamille ist demgegenüber das Gewicht der praktisch wasserfreien Droge. Ist zum Beispiel das Verhältnis von Frischkamille zu Extraktionsmittel hoch, so muß der Alkoholgehalt des Extraktionsmittels höher sein. Es soll jedoch 1 Gewichtsteil Trockengewicht der Frischkamille (bestimmt durch 3 stündiges Trocknen der Probe bei 105°C im Trockenschrank) mindestens 4 Gewichtsteilen, vorzugsweise 4—8 Gewichtsteilen, insbesondere 5 bis 6 Gewichtsteilen Extrakt entsprechen, wobei der Alkoholgehalt des Extraktionsmittels so zu bemessen ist, daß im Extrakt ein Alkoholgehalt von 20 Gewichts% nicht unterschritten wird. Im allgemeinen soll der Alkoholgehalt des Extraktes 20 bis 50, vorzugsweise 20 bis 40 Gewichts%, insbesondere 20 bis 35 Gewichts% betragen. 100 g Frischkamille entsprechen im allgemeinen 20—30 g Trockengewicht. Je nach Alkoholgehalt des Extraktes kommen zum Beispiel auf 100 g Frischkamille folgende Alkoholmengen in Abhängigkeit vom Alkoholgehalt in Betracht (wobei das Trockengewicht der Frischkamille beispielsweise 24,6 g ist, entsprechend einem Wassergehalt von 73,6 Gewichts%): 57 g Ethanol (96 Volumenprozent) auf 100 g einer solchen Frischkamille ergeben beispielsweise einen Alkoholgehalt von 40 Gewichts% im Extrakt.

Die Wirkstoffausbeute ist analog zur Drogenextraktion abhängig vom Alkoholgehalt des Extraktionsmediums. Das an die Frischkamille gebundene Wasser (=Wassergehalt) stellt zusammen mit dem zugesetzten Alkohol das Extraktionsmedium dar. Unter Berücksichtigung des Wassergehalts der Frischkamille ist daher der Alkoholzusatz so zu wählen, daß im Extrakt der angestrebte Alkoholgehalt resultiert.

Unter frischen Kamillenblüten (Frischkamille) werden Kamillenblüten verstanden, die innerhalb von 24 Stunden nach dem Pflücken extrahiert werden oder innerhalb diese Zeit eingefroren werden. Hierbei findet innerhalb dieser 24 Stunden nur eine mäßige Trocknung von maximal 10% (bezogen auf den Wassergehalt der frischen Kamillenblüten) entsprechend den klimatischen Verhältnissen der Außenwelt (Temperatur, Luftfeuchtigkeit) statt.

In der Regel werden die Kamillenblüten in den ersten 24 Stunden nach der Pflücke extrahiert. Ist eine Extraktion innerhalb dieses Zeitraumes nicht durchführbar, so kann die Kamille bis zur Extraktion eingefroren werden.

Für das erfindungsgemäße Verfahren können auch eingefrorene Kamillenblüten verwendet werden sofern für das Einfrieren frische Kamillenblüten (wie oben definiert) verwendet werden.

Das Einfrieren der frischen Kamillenblüten kann beispielsweise in folgender Weise erfolgen:

Die frische Kamille wird beispielsweise mit kalter Luft, bei −10 bis −50°C, insbesondere −30 bis −50°C, vorzugsweise −35 bis −45°C oder mit flüssigen Gasen, chargenweise oder kontinuierlich eingefroren und bei Temperaturen zwischen beispielsweise −10 bis −50°C, insbesondere −30 bis −50°C (vorzugsweise −35 bis −45°C) gelagert.

Das kontinuierliche Tiefgefrieren mit flüssigen Gasen kann beispielsweise mit Kohlendioxid, Stickstoff oder Frigenen vorzugsweise mit Flüssigstickstoff, in einem Tunnelfroster vorgenommen werden. Die Lagerung erfolgt bei den Temperaturen die vorstehend angegeben sind. Für die Extraktion können die tiefgefrorenen Kamillenblüten direkt verwendet werden oder sie werden in schonender Weise ganz oder teilweise aufgetaut (beispielsweise durch Übergießen mit dem 30—60°C warmen Extraktionsmittel).

Wenn statt Kamillendroge Frischkamille extrahiert wird, verbessert sich die Wirkstoffausbeute. So wurden auf der Basis gleichen Trockengewichts und gleichen Drogenäquivalents (1 Teil Trockengewicht=5 Teile Extrakt) und einem Ethanolgehalt von ca. 33% im Extrakt folgende Wirkstoffausbeuten, bezogen auf den Gehalt der Frischkamille (=100%) und berechnet auf deren Trockengewicht gefunden:

|  | Frischkamillen-extraktion | Drogen-extraktion |
|---|---|---|
| Ätherisches Öl | 54,6% | 39,5% |
| Chamazulen | 100% | 24,1% |
| (−)-α-Bisabolol | 58,8% | 26,7% |

Die Trocknung der Frischkamille wurde für diesen Vergleich unter schonenden Bedingungen durchgeführt. Diese schonenden Bedingungen sind jedoch in der Produktionspraxis nicht nachvollziehbar. Bei einem Vergleich mit einer Droge, die gemäß der üblichen Produktionspraxis erhalten wird, würde daher die erfindungsgemäße Frischkamillenextraktion einen nochmals verbesserten Wirkungsgrad ergeben.

Bei der Frischkamillenextraktion entfällt also nicht nur der durch die Trocknung der Kamillenblüten verursachte Wirkstoffverlust, auch die Extraktionsausbeute, besonders die an Chamazulen und (−)-α-Bisabolol, wird verbessert. Die höhere Wirkstoffausbeute gestattet es überdies, eine krautreichere Kamille zu verwenden. Eine Absiebung der bei der Blütenpflücke zwangsläufig anfallenden Krautanteile ist dann nicht mehr erforderlich.

Durch die Frischkamillenextraktion wird nicht nur die Benutzung kostenaufwendiger Trocknungsanlagen überflüssig. Auch die mit ihrem Betrieb verbundenen erheblichen Energiekosten entfallen. Die Kosten für die Extraktherstellung lassen sich auf diese Weise deutlich senken.

Wie Versuche gezeigt haben, können bei der Extraktion von Frischkamille auch wirkstoffreiche Kamillenextrakte hergestellt werden mit niedrigerem Alkoholgehalt (zum Beispiel mit einem Gehalt von 25% Alkohol) als dem in der Literatur empfohlenen Gehalt von 45%. Überraschenderweise stellte sich heraus, daß selbst in Frischkamillenextrakten mit einem Alkoholgehalt von 25% keine enzymatische Veränderung der Wirkstoffe stattfindet. Durch die Senkung des Alkoholgehaltes auf 25% erhält man einen Extrakt, der im Gesamtgehalt an Extraktivstoffen (wasserlöslichen und wasserunlöslichen) um ca. 20% höher liegt als ein Extrakt aus Kamillendroge mit einem Alkoholgehalt von 45%.

Schließlich hat sich gänzlich unerwartet herausgestellt, daß die Haltbarkeit der Wirkstoffe in den Frischkamillenextrakten erheblich besser ist als in den Kamillendrogenextrakten. So war beispielsweise bei Extrakten aus Frischkamille mit 26% beziehungsweise 40% Ethanolgehalt nach 9 Monaten Lagerdauer bei Raumtemperatur kein Rückgang an Matricin beziehungsweise Chamazulen festzustellen. Ein solches Verhalten war um so weniger zu vermuten, als durch die Art der Extraktion eher ein verstärkter Abbau durch enzymatische Aktivität erwartet werden mußte. Aus der Literatur ist bekannt, daß besonders das Chamazulen, das in der Kamille selbst und in den Kamillenextrakten in Form der Vorstufe Matricin vorliegt,

# EP 0 175 185 B1

während der Lagerung einen raschen Abbau erleidet. Bischer ist kein Verfahren bekannt geworden, das die Stabilität des Matricins in Kamillenextrakten nachhaltig zu sichern in der Lage ist.

## TABELLE 1
### Wirkstoffgehalt in Kamillenextrakten nach 9 Monaten
### (Ausgangswert=100)

| | Drogen-extraktion | Frischkamillenextraktion | |
|---|---|---|---|
| Alkoholgehalt im Extrakt | 40% | 40% | 25% |
| Ätherisches Öl | 86% | 91% | 86% |
| Chamazulen | 27% | 130% | 109% |
| (−)-α-Bisabolol | 98% | 102% | 83% |

Wie aus der Tabelle 1 hervorgeht, sinkt der Azulengehalt in dem aus Kamillendroge hergestellten Extrakt nach einer Lagerung von 9 Monaten erwartungsgemäß drastisch ab, während der Gehalt an ätherischem Öl und Bisabolol relativ stabil bleibt. Bei den Frischkamillenextrakten verhält sich der Gehalt an ätherischem Öl und Bisabolol vergleichbar dem Drogenextrakt. Dagegen ist der Chamazulengehalten erstaunlich stabil geblieben, gegenüber dem Ausgangswert sogar leicht angestiegen.

Bei der Frischkamillenextraktion kann man von den bekannten Extraktionsverfahren ausgehen. Da die Frischkamille voluminöser als die Kamillendroge ist, ist es jedoch zweckmäßig, sich vorzugsweise des Verfahrens der Mazeration beziehungsweise der bewegten Mazeration zu bedienen, beispielsweise des in der deutschen Patentschrift 1 093 951 beschriebenen Verfahrens.

Das erfindungsgemäße Verfahren ist ganz allgemein anwendbar, das heißt es eignet sich für alle vorkommenden Kamillen. Besonders geeignet ist das Verfahren bei Verwendung der Kamillensorte Degumill (DDR-Sortenschutzrecht Degumill; Deutsches Patent 24 02 802; Italienisches Patent 1 035 096), der Kamillensorte Manzana (Deutsche Offenlegungsschrift 34 23 207) sowie von Kamillen gemäß der deutschen Patentanmeldung P 34 46 216.3.

So erhalten erfindungsgemäße Frischkamillenauszüge von Kamillenblüten der Sorte Degumill beispielsweise mindestens 18 mg% (−)-α-Bisabolol und weniger als 9 mg% an übrigen Bisaboloiden (wie Bisabololoxide).

Bei Verwendung von Kamillenblüten der Sorte Manzana oder von Kamillenblüten gemäß der deutschen Patentanmeldung P 34 46 216.3 enthalten die erfindungsgemäßen Kamillenauszüge beispielsweise mindestens 24 mg% (−)-α-Bisabolol und weniger als 12 mg% an übrigen Bisaboloiden.

Da frische Kamillenblüten im allgemeinen noch die zugehörigen Flügelblätter vollständig oder zumindest teilweise enthalten, weisen die erfindungsgemäßen Extrakte auch einen höheren Flavongehalt auf als die bisher aus getrockneten Blüten hergestellten Extrakte. Selbstverständlich ist es auch möglich bei der erfindungsgemäßen Extraktion zusätzlich frische Kamillenflügelblätter, eingefrorene Kamillenflügelblätter oder auch getrocknete Kamillenflügelblätter zuzusetzen, wodurch der Flavongehalt zusätzlich erhöht werden kann. Die Kamillenflügelblätter weisen einen hohen Gehalt an Flavonen auf (wie zum Beispiel Apigenin und dessen Glykoside).

Beispiel 1

Eine 200 g Kamillendroge äquivalente Menge an Frischkamille (758 g, Wassergehalt 73,6%; ätherisches Öl 876 mg%; Azulen 57 mg%; Bisabolol 151 mg%) wird mit 510 g Ethanol (84 Gewichts%) in einem Muldenmischer bei einer Drehzahl des Mischwerks von 65 Umdrehungen pro Minute extrahiert. Nach 30 Minuten wird das Extraktionsgut abgepreßt und der Extrakt filtriert. Im Extrakt wird der Wirkstoffgehalt in bekannter Weise ermittelt:

| | |
|---|---|
| Azulen | 14,7 mg% |
| ätherisches Öl | 105,7 mg% |
| Bisabolol | 20,4 mg% |
| Apigenin und Apigeninglucoside (berechnet als Apigenin) | 73,1 mg% |
| Extraktivstoffe | 5,9% |
| Ethanol | 39,6% (g/g) |

Vergleichsbeispiel (bekanntes Verfahren)

200 g der wie oben verwendeten, nun jedoch getrockneten Kamillenblüten (die Trocknung erfolgt unter

4

Ausschluß des direkten Sonnenlichts in dünner Schicht bei Temperaturen unter 40°C; ätherisches Öl 598 mg%; Azulen 22,7 mg%; Bisabolol 150 mg%; Wassergehalt 9%) werden mit 1050 g Ethanol (40 Gewichts%) in einem Muldenmischer bei einer Drehzahl des Mischwerks von 65 Umdrehungen pro Minute extrahiert. Nach 30 Minuten wird das Drogengut abgepreßt und der Extrakt filtriert. Im Extrakt wird der Wirkstoffgehalt in bekannter Weise ermittelt:

| | |
|---|---|
| Azulen | 4,7 mg% |
| ätherisches Öl | 87,0 mg% |
| Bisabolol | 12,9 mg% |
| Apigenin und Apigeninglucoside (berechnet als Apigenin) | 59,9 mg% |
| Extraktivstoffe | 5,3 mg% |
| Ethanol | 37,9% (g/g) |

Beispiel 2

Eine 200 g Kamillendroge äquivalente Menge an Frischkamille (952 g; Wassergehalt 79%; ätherisches Öl 876 mg%; Azulen 57 mg%, Bisabolol 151 mg%) wird mit 540 g Ethanol (80 Gewichtsprozent) in einem Muldenmischer bei einer Drehzahl des Mischwerkes von 65 Umdrehungen pro Minute extrahiert. Nach 30 Minuten wird das Extraktionsgut abgepreßt und der Extrakt filtriert. Im Extrakt wird der Wirkstoffgehalt in bekannter Weise ermittelt:

| | |
|---|---|
| Azulen | 8,2 mg% |
| ätherisches Öl | 75,5 mg% |
| Bisabolol | 9,1 mg% |
| Apigenin und Apigeninglucoside (berechnet als Apigenin) | 62,2 mg% |
| Extraktivstoffe | 6,6% |
| Ethanol | 26,5% (g/g) |

Vergleichsbeispiel (bekanntes Verfahren)

200 g der wie vorstehend verwendeten, nun jedoch getrockneten Kamillenblüten (Trocknung wie bei Vergleichsbeispiel 1; ätherisches Öl 598 mg%; Azulen 22,7 mg%; Bisabolol 150 mg%; Wassergehalt 9%) werden mit 1050 g Ethanol (25 Gewichtsprozent) in einem Muldenmischer bei einer Drehzahl des Mischwerks von 65 Umdrehungen pro Minute extrahiert. Nach 30 Minuten wird das Drogengut abgepreßt und der Extrakt filtriert. Im Extrakt wird der Wirkstoffgehalt in bekannter Weise ermittelt:

| | |
|---|---|
| Azulen | 3,6 mg% |
| ätherisches Öl | 66,0 mg% |
| Bisabolol | 6,7 mg% |
| Apigenin und Apigeninglucoside (berechnet als Apigenin) | 48,9 mg% |
| Extraktivstoffe | 6,3 mg% |
| Ethanol | 25,4% (g/g) |

Beispiel 3

Eine 300 g Kamillendroge äquivalente Menge an Frischkamille (1154 g; Wassergehalt 74%; ätherisches Öl 950 mg%; Azulen 92 mg%; Bisabololoxid A 180 mg%) wird mit 1330 g Propanol-(2) (52 Gewichtsprozent) in einem Muldenmischer bei 65 Umdrehungen pro Minute extrahiert. Nach 90 Minuten wird das Extraktionsgut abgepreßt und der Extrakt filtriert. Im Extrakt wird der Wirkstoffgehalt in bekannter Weise ermittelt:

| | |
|---|---|
| Azulen | 14,2 mg% |
| ätherisches Öl | 101,5 mg% |
| Bisabololoxid A | 26,0 mg% |
| Apigenin und Apigeninglucoside (berechnet als Apigenin) | 57,0 mg% |
| Extraktivstoffe | 6,9 mg% |
| Propanol-(2) | 26,0% (g/g) |

Vergleichsbeispiel (bekanntes Verfahren)

300 g der wie vorstehend verwendeten, nun jedoch getrockneten Kamillenblüten (Trocknung wie bei Vergleichsbeispiel 1; ätherisches Öl 740 mg%; Azulen 56 mg%; Bisabololoxid A 168 mg%; Wassergehalt 9%) werden mit 2100 g Propanol-(2) (33 Gewichtsprozent) in einem Muldenmischer bei 65 Umdrehungen pro Minute extrahiert. Nach 90 Minuten wird das Drogengut abgepreßt und der Extrakt filtriert. Im Extrakt wird der Wirkstoffgehält in bekannter Weise ermittel:

# EP 0 175 185 B1

| Azulen | 6,7 mg% |
|---|---|
| ätherisches Öl | 85,2 mg% |
| Bisabololoxid A | 17,4 mg% |
| Apigenin und Apigeninglucoside | |
| (berechnet als Apigenin) | 52,4 mg% |
| Extraktivstoffe | 6,6 mg% |
| Propanol-(2) | 28,5% (g/g) |

## Patentansprüche

1. Verfahren zur Gewinnung haltbarer wirkstoffreicher Kamillenextrakte ohne nachträgliche Hitzebehandlung, dadurch gekennzeichnet, daß frische Kamillenblüten oder eingefrorere Kamillenblüten mit wässrigen $C_1$—$C_4$-Alkanolen, deren Alkanolgehalt zwischen 40—100% liegt, in einem Temperaturbereich zwischen 10 und 60°C extrahiert werden.

2. Verfahren nach Anspruch 1 dadurch gekennzeichnet, daß vor oder während der Extraktion zusätzlich frische, eingefrorene oder getrocknete Kamillenblütenblätter zugestzt werden.

3. Haltbare wirkstoffreiche Kamillenextrakte erhältlich durch Extraktion in einem Temperatur bereich zwischen 10 und 60°C von frischen oder eingefrorenen Kamillenblüten mit wässrigen $C_1$—$C_4$-Alkanolen deren Alkanolgehalt zwischen 40—100% liegt.

4. Haltbare wirkstoffreiche Kamillenextrakte erhältlich nach Anspruch 3, die mindestens 18 mg% (−)-α-Bisabolol und weniger als 9 mg% an übrigen Bisaboloiden enthalten.

5. Haltbare wirkstoffreiche Kamillenextrakte mit erhötem Flavongehalt erhältlich nach Anspruch oder 4 mit unter Zusatz von frischen, eingefrorenen oder getrockneten Flügelblättern.

6. Kamillenextrakte, nach einem oder mehreren der vorangegangenen Ansprüche.

7. Verwendung von frischen oder eingefrorenen Kamillenblüten für die direkte Herstellung von Kamillenextrakten durch Extraktion in einem Temperatur bereich zwischen 10 und 60°C mit wässrigem $C_1$—$C_4$-Alkanolen (Alkanolgehalt zwischen 40—100%), gegebenenfalls unter Zusatz von frischen, eingefrorenen oder getrockneten Kamillenflügelblättern.

## Revendications

1. Procédé pour l'obtention d'extraits de camomille riches en principes actifs et conservables, sans traitement supplémentaire par la chaleur, caractérisé en ce que des fleurs de camomille fraîches ou des fleurs de camomille congelées sont extraites dans une gamme de température comprise entre 10 et 60°C avec des alcanols en $C_1$—$C_4$ aqueux dont la teneur en alcanol se situe entre 40 et 100%.

2. Procédé selon la revendication 1, caractérisé en ce que des pétales de camomille frais, congelés ou séchés sont ajoutés en plus avant ou pendant l'extraction.

3. Extraits de camomille riches en principes actifs et conservables, obtenus par extraction, dans une gamme de température comprise entre 10 et 60°C, de fleurs de camomille fraîches ou congelées avec des alcanols en $C_1$—$C_4$ aqueux dont la teneur en alcanol se situe entre 40 et 100%.

4. Extraits de camomille riches en principes actifs et conservables, obtenus selon la revendication 3, qui contiennent au moins 18 mg% de (−)-α-bisabolol et moins de 9 mg% d'autres bisaboloïdes.

5. Extraits de camomille riches en principes actifs et conservables, à teneur accrue en flavone, obtenus selon la revendication 3 ou 4 avec addition de pétales frais, congelés ou séchés.

6. Extraits de camomille selon l'une quelconque des revendications 3 à 5.

7. Utilisation de fleurs de camomille fraîches ou congelées pour la préparation directe d'extraits de camomille par extraction dans une gamme de température comprise entre 10 et 60°C avec des alcanols en $C_1$—$C_4$ aqueux (teneur en alcanol, entre 40 et 100%), éventuellement avec addition de pétales de camomille frais, congelés ou séchés.

## Claims

1. A process for obtaining stable camomile extracts rich in active substance without subsequent heat treatment, characterized in that fresh camomile flowers or frozen camomile flowers are extracted with aqueous $C_1$—$C_4$-alkanols, the alkanol content of which lies between 40—100% in a temperature range between 10 and 60°C.

2. A process according to Claim 1, characterized in that additional fresh, frozen or dried camomile flower petals are added before or during extraction.

3. Stable camomile extracts rich in active substance obtainable by extraction of fresh or frozen camomile flowers in a temperature range between 10 and 60°C with aqueous $C_1$—$C_4$-alkanols, the alkanol content of which lies between 40—100°C.

4. Stabile camomile extracts obtainable as set out in Claim 3 which contain at least 18 mg% (−)-α-bisabolol and less than 9 mg% of other bisaboloids.

5. Stabile camomile extracts rich in active substance with an elevated flavone content obtainable according to Claim 3 or Claim 4 with addition of fresh, frozen or dried wing petals.

6

6. A camomile extract according to one or several of the preceding Claims.

7. The use of fresh or frozen camomile flowers for the direct preparation of camomile extracts by extraction in a temperature range between 10 and 60°C with aqueous $C_1$—$C_4$-alkanols (alkanol content between 40—100°C), optionally with the addition of fresh, frozen or dried camomile wing petals.